# EUROPEAN PATENT APPLICATION

(11) **EP 4 790 144 A1**
(43) Date of publication of application: **12.08.2026**
(21) Application number: 24876226.2
(22) Date of filing: 09.08.2024
(51) Int. Cl.: C12Q 1/68, G01N 33/74

(54) **USE OF NOVEL BIOMARKER IN DIAGNOSING OR ASSISTING IN DIAGNOSING SPN**

(30) Priority: 11.10.2023 CN 202311315347
(71) Applicant: Peking Union Medical College Hospital, Beijing 100730 (CN)
(72) Inventor: PENG, Junya, Beijing 100730 (CN); WU, Wenming, Beijing 100730 (CN); WANG, Wenze, Beijing 100730 (CN); LIU, Yingao, Beijing 100730 (CN); LIU, Yuanhao, Beijing 100730 (CN); ZHAO, Yupei, Beijing 100730 (CN)
(74) Representative: Wichmann, Hendrik
(86) International application number: PCT/CN2024/111170
(87) International publication number: WO 2025/077406

(57) **Abstract**

The present application relates to the use of a novel biomarker in diagnosing or assisting in diagnosing SPN, in particular to the use of ABCD1 as a novel marker in diagnosing or assisting in diagnosing SPN. The present application further relates to the use of ABCD1 as a novel marker in the development of a reagent for diagnosing or assisting in diagnosing SPN.

## Description

### TECHNICAL FIELD

The present application belongs to the field of biomedicine, and relates to the use of ABCD1 as a novel biomarker in the development of reagents for diagnosing or assisting in the diagnosis of solid pseudopapillary neoplasm of the pancreas (SPN).

### BACKGROUND

Tumors refer to newly formed neoplastic growths arising from the proliferation of local tissue cells under the influence of various tumorigenic factors in the body. Such growths typically present as space-occupying mass-like protrusions, and are therefore also termed neoplasms. According to the biological behavior and their degree of harm to the organism, tumors can be classified into benign and malignant categories. Benign tumors are generally characterized by slow growth, well-circumscribed boundaries, and a lack of metastatic potential, and thus usually exert limited detrimental effects on human health. In contrast, malignant tumors exhibit rapid proliferation, possess the capacity for local invasion and distant metastasis, and may produce deleterious factors that disrupt normal tissue architecture, impair organ function, and ultimately pose a significant threat to life.

Solid pseudopapillary neoplasm of the pancreas (SPN) is a rare low-grade malignant tumor, which accounts for 0.9-2.7% of all pancreatic tumors. SPN predominantly occurs in females aged 20-30, with a lower incidence in males. It is generally characterized by an indolent clinical course and slow progression, and is asymptomatic in most cases. However, a subset of patients may present with non-specific symptoms, including abdominal discomfort, right upper quadrant tenderness, and diarrhea. In some cases, the tumor growth may exert mass effects on adjacent organs, leading to pancreatitis, hypersplenism, and gallbladder hydrops, etc.

The diagnosis of SPN is primarily established based on a combination of imaging findings and histopathological features. The imaging evaluation represents the first-line modality for the identification of SPN. Commonly utilized imaging techniques include: abdominal ultrasound, abdominal computed tomography (CT), and magnetic resonance imaging (MRI), etc. According to the guidelines of the International Association of Pancreatology (IAP), the diagnosis of SPN is generally supported by the following criteria: (1) imaging examinations show that the lesion is a pancreatic parenchymal lesion; (2) the lesion is usually between 2 cm and 10 cm in diameter; (3) the lesion has both cystic and solid structures; (4) the lesion is well-demarcated; and (5) the lesion exhibits significant enhancement on contrast-enhanced imaging. In clinic practice, a definitive diagnosis of SPN and its distinction from other pancreatic neoplasms rely on histological examination. The typical histological features of SPN include: (1) tumor cells are arranged in cystic or pseudopapillary patterns; (2) the cystic spaces are filled with mucinous or hemorrhagic material; (3) the presence of fibrous stroma and lipid deposition between tumor cells and cystic structures; and (4) the tumor cells show a relatively heterogeneous morphology, with enlarged nuclei, conspicuous nuclear atypia, and few or absent mitotic figures. SPN is characterized by a positive multilineage expression profile, including epithelial, mesenchymal and neuroendocrine markers, mainly comprising: (1) β-catenin: which typically exhibits aberrant nuclear accumulation in SPN tumor cells, with a positivity rate of up to 95%, and serves as a key marker in the differential diagnosis of SPN from other tumors; (2) CD10: CD10 shows a high rate of positivity in SPN and can be used to distinguish SPN from other tumors such as pancreatic neuroendocrine tumors and pancreatic ductal adenocarcinoma; (3) vimentin: vimentin is a mesenchymal marker that is consistently expressed in SPN tumor cells but is generally absent in other pancreatic tumor types; and (4) α1-antitrypsin, which demonstrates a high positivity rate in SPN tumor cells but is typically not expressed in other neoplasms.

SPN typically exhibits characteristic histomorphological features, however, some SPN cases may be confused with other tumors, a typical example being non-functional pancreatic neuroendocrine tumor (NF-PanNET). NF-PanNET is a relatively rare type of pancreatic neoplasms, accounting for about 5%-10% of all the pancreatic tumors, and is characterized by the absence of significant hormone hypersecretion. Both SPN and NF-PanNET are generally large, well-circumscribed intrapancreatic masses, which may be solid or mixed solid-cystic and may show hemorrhage, calcification and necrosis. In certain instances, SPN and NF-PanNET share overlapping morphological features. Under microscopic observation, tumor cells in both may be arranged in solid, trabecular or acinar patterns. The tumor cells are often relatively uniform in size and are often closely arranged as round or polygonal cells, with a relatively low nuclear-to-cytoplasmic ratio, rendering morphological distinction challenging. Furthermore, SPN and NF-PanNET may exhibit similar immunohistochemical staining results, with variable expression of markers including vimentin, synapsin, CD200, CD99, Syn and NSE. Under certain conditions, the clinical manifestations of SPN and NF-PanNET may be similar. Both may occur in young female patients, are frequently detected incidentally, and may be asymptomatic or present only with non-specific symptoms such as abdominal pain and upper abdominal discomfort, and may lack hormone hypersecretion. At the same time, their imaging features may also be similar. Therefore, an accurate diagnosis of SPN versus NF-PanNET requires integrated evaluation of clinical manifestations, imaging findings, and pathological examinations.

SPN shares overlapping morphological features with a variety of other pancreatic tumors (including non-functional pancreatic neuroendocrine tumors (NF-PanNET), acinar cell carcinoma, pancreatoblastoma, etc.). In the absence of typical clinical and histomorphological manifestations, immunohistochemistry plays an important role in the differential diagnosis between SPN and other tumor types. Although β-catenin is widely regarded as an important marker for the differential diagnosis of SPN, it typically shows nuclear accumulation and presents as nuclear positivity. However, β-catenin is not indispensable in the differential diagnosis of SPN and other tumors. Previous studies have reported β-catenin is also positively expressed in pancreatic neuroendocrine tumors and pancreatoblastoma. At present, no immunohistochemical profile is entirely specific for SPN, and existing immunohistochemical markers show varying degrees of overlapping expression among the above-mentioned.

Furthermore, under physiological conditions, wild-type β-catenin may also exhibit a certain degree of nuclear localization. In addition, diffuse cytoplasmic expression of β-catenin may be observed, which can interfere with the interpretation of nuclear staining and may lead to false-positive or false-negative diagnostic results. Both in primary and metastatic lesions of SPN, when the histomorphological features are similar, relying solely on currently available immunohistochemical markers remains insufficient to achieve an accurate diagnosis. In practice, evaluation incorporating clinical, imageology, and pathological features is required.

In summary, SPN shares a certain degree of overlap with multiple pancreatic neoplasms, including NF-PanNET, acinar cell carcinoma, and pancreatoblastoma, in terms of gross appearance, histomorphological features, and immunohistochemical staining patterns. For cases in which the differential diagnosis of SPN is challenging, there remains an unmet need for novel, highly specific pathological biomarkers to enable a definitive diagnosis.

### SUMMARY

To solve the above-mentioned technical problems, the present application provides a novel marker, i.e., ATP-binding cassette sub-family D member 1 (ABCD1), which is used for diagnosing or assisting in the diagnosis of SPN. In some embodiments, the marker ABCD1 can be used for differentiating or assisting in the differentiation of SPN from other non-SPN tumors. In some embodiments, the marker ABCD1 can be used for screening or assisting in the screening of SPN patients.

In one aspect, the present application provides an antibody binding to ATP-binding cassette sub-family D member 1 (ABCD1) or an antigen-binding fragment thereof, wherein the complementarity-determining regions (CDRs) have the following amino acid sequences: a heavy chain CDR1 selected from at least one of amino acid sequences set forth in SEQ ID NOs: 1-8; a heavy chain CDR2 selected from at least one of amino acid sequences set forth in SEQ ID NOs: 9-17; a heavy chain CDR3 selected from at least one of amino acid sequences set forth in SEQ ID NOs: 18-24; a light chain CDR1 selected from at least one of amino acid sequences set forth in SEQ ID NOs: 25-30; a light chain CDR2 selected from at least one of amino acid sequences LVS, WAS, RMS or YAS; and a light chain CDR3 selected from at least one of amino acid sequences set forth in SEQ ID NOs: 31-35.

In one aspect, the present application provides a polynucleotide, wherein the polynucleotide encodes the antibody or the antigen-binding fragment thereof according to the present application.

In one aspect, the present application provides a recombinant vector, wherein the recombinant vector comprises the polynucleotide according to the present application.

In one aspect, the present application provides a host cell, wherein the host cell comprises the polynucleotide according to the present application and/or the recombinant vector according to the present application.

In one aspect, the present application provides a pharmaceutical composition, wherein the pharmaceutical composition comprises the antibody or the antigen-binding fragment thereof according to the present application.

In one aspect, the present application provides a kit, wherein the kit comprises the antibody or the antigen-binding fragment thereof according to the present application.

In one aspect, the present application provides a reagent or kit comprising a substance for detecting ABCD1, wherein the reagent or kit is used for any one or more of the following (a)-(c): (a) the reagent or kit is used for diagnosing or assisting in the diagnosis of SPN; (b) the reagent or kit is used for differentiating or assisting in the differentiation of SPN from other non-SPN tumors; and/or (c) the reagent or kit is used for screening or assisting in the screening of SPN patients.

In another aspect, the present application provides a system for diagnosing or assisting in the diagnosis of SPN, comprising a detection system and an interpretation system, wherein the detection system is used for ABCD1 immunostaining on tumor tissues or tumor cells in a subject; and the interpretation system is used for converting ABCD1 immunostaining results into interpretation results which refer to SPN or non-SPN.

In yet another aspect, the present application provides a system for differentiating or assisting in the differentiation of SPN from other non-SPN tumors, comprising a detection system and an interpretation system, wherein the detection system is used for ABCD1 immunostaining of tumor tissues or tumor cells in a subject; and the interpretation system is used for converting ABCD1 immunostaining results into interpretation results which refer to SPN or other non-SPN tumors.

In one aspect, the present application provides use of ABCD1 as a detection marker in any one or more of the following (a)-(c): (a) use in the development of a diagnostic reagent for diagnosing or assisting in the diagnosis of SPN; (b) use in the development of a differentiation reagent for differentiating or assisting in the differentiation of SPN from other non-SPN tumors; and/or (c) use in the development of a screening reagent for screening or assisting in the screening of SPN patients.

In yet another aspect, the present application provides use of a substance for detecting ABCD1 in any one or more of the following (a)-(c): (a) use in the preparation of a diagnostic reagent for diagnosing or assisting in the diagnosis of SPN; (b) use in the preparation of a differentiation reagent for differentiating or assisting in the differentiation of SPN from other non-SPN tumors; and/or (c) use in the preparation of a screening reagent for screening or assisting in the screening of SPN patients.

In still another aspect, the present application provides use of a substance for detecting ABCD1 in the preparation of a kit, wherein the kit is configured for any one or more of the following (a)-(c): (a) for diagnosing or assisting in the diagnosis of SPN; (b) for differentiating or assisting in the differentiation of SPN from other non-SPN tumors; and/or (c) for screening or assisting in the screening of SPN patients.

In one aspect, the present application provides a method for diagnosing or assisting in the diagnosis of SPN, comprising: using ABCD1 as a marker and detecting the marker for diagnosing or assisting in the diagnosis of SPN.

In another aspect, the present application provides a method for differentiating or assisting in the differentiation of SPN from other non-SPN tumors, comprising: using ABCD1 as a marker and detecting the marker for differentiating or assisting in the differentiation of SPN from other non-SPN tumors.

In yet another aspect, the present application provides a method for screening or assisting in the screening of SPN patients, comprising: using ABCD1 as a marker and detecting the marker for screening or assisting in the screening of SPN patients.

### BRIEF DESCRIPTION OF THE DRAWINGS

FIG. 1 shows representative transmission electron microscopy (TEM) images and statistical results from Step 1 of Example 1.
FIG. 2 shows representative immunofluorescence staining images and statistical results from Step 1 of Example 1.
FIG. 3 shows differential gene expression results presented by volcano plots in Step 2 of Example 1, comparing pancreatic tumor tissues and adjacent normal pancreatic tissues of SPN patients, as well as pancreatic tumor tissues of SPN patients and pancreatic tumor tissues of NF-PanNET patients.
FIG. 4 shows western blotting results of pancreatic tumor tissues and adjacent normal pancreatic tissues of SPN patients in Step 2 of Example 1.
FIG. 5 shows representative images of hematoxylin-eosin (HE) staining of paraffin sectionsand immunohistochemical (IHC) staining for ABCD1 of pancreatic tumor tissues and adjacent normal pancreatic tissues of SPN patients in Step 2 of Example 1.
FIG. 6 shows a representative image of each score in Example 2.
FIG. 7 shows the scoring results of pancreatic tumor tissues and adjacent normal pancreatic tissues from 50 SPN patients, as well as the scoring results of metastatic tumor tissues from 16 SPN patients in Example 2.
FIG. 8 shows a receiver operating characteristic (ROC) curve in Step 2 of Example 3.
FIG. 9 shows a ROC curve in Step 3 of Example 3.
FIG. 10 shows representative IHC staining images of pancreatic tumor tissues from patients with pancreatic neuroendocrine tumors in Example 4.
FIG. 11 shows representative IHC staining images of pancreatic tumor tissues from patients with pancreatic acinar cell carcinoma in Example 4.
FIG. 12 shows representative IHC staining images of pancreatic tumor tissues from patients with pancreatoblastoma in Example 4.
FIG. 13 shows representative IHC staining images of pancreatic tumor tissues from patients with pancreatic ductal adenocarcinoma in Example 4.
FIG. 14 shows the scoring results of 99 SPN patients in Example 3, 91 NF-PanNET patients in Example 4, 10 patients with insulinoma in Example 4, 9 patients with acinar cell carcinoma in Example 4, 3 patients with pancreatoblastoma in Example 4, and 10 patients with pancreatic ductal adenocarcinoma in Example 4.
FIG. 15 and FIG. 16 show the ELISA results of the binding activities of the antibodies Ms 602-5-2, Ms 602-5-4, Ms 602-5-8, Ms 601-C-3, Ms 601-C-8, Ms 601-C-9, Ms 601-C-17, Ms 601-C-27, Ms 601-C-28 and Ms 601-C-44 prepared in Example 5 to ABCD1 protein.
FIG. 17 shows the scoring results obtained using the antibodies prepared in Example 5 for 30 cases of solid pseudopapillary neoplasm of the pancreas, 20 cases of pancreatic neuroendocrine tumor, 10 cases of pancreatic intraepithelial neoplasia, 20 cases of pancreatic ductal adenocarcinoma, 4 cases of serous cystadenoma, 4 cases of mucinous cystadenoma, 3 cases of acinar cell carcinoma, and 3 cases of pancreatoblastoma in Example 6. In the figure, the vertical axis represents the percentage of samples with each score relative to the total number of samples in each group.
FIG. 18 shows representative IHC staining images obtained using the antibodies prepared in Example 5 for solid pseudopapillary neoplasm of the pancreas, pancreatic neuroendocrine tumor, pancreatic intraepithelial neoplasia, pancreatic ductal adenocarcinoma, serous cystadenoma, mucinous cystadenoma, acinar cell carcinoma and pancreatoblastoma tissues in Example 6.

### DETAILED DESCRIPTION OF THE EMBODIMENTS

Unless stated otherwise, the terms used herein have the ordinary meaning as commonly understood by a person skilled in the art. For a person skilled in the art, the parameters may vary depending on the desired properties and effects intended in the present application. Each numerical parameter should be interpreted in view of the number of significant digits and conventional rounding methods, or in a manner understood by a person skilled in the art. Generally, the nomenclature used herein and the experimental procedures described herein for organic chemistry, medicinal chemistry, and biology are well known and commonly used in the art. Unless otherwise defined, all technical and scientific terms used herein generally have the same meaning as commonly understood by one of ordinary skill in the art to which this application belongs. If multiple definitions exist for terms used herein, the definitions set forth in this section shall prevail unless otherwise stated.

Herein, "comprising", "containing", "including", "having" and the like should be understood as open-ended, meaning "including but not limited to", and encompass not only the explicitly recited components but also other components. The phrases "consisting of" and "composed of" are closed-ended, and include only the explicitly recited components.

As used herein, the expression "A and/or B" includes the following three scenarios: (1) A; (2) B; and (3) A and B. The expression "A, B, and/or C" includes the following seven scenarios: (1) A; (2) B; (3) C; (4) A and B; (5) A and C; (6) B and C; and (7) A, B, and C. The meaning of similar expressions is analogous.

As used herein, ABCD1 stands for ATP-binding cassette sub-family D member 1. The ABCD1 gene maps to the long arm of the human X chromosome (Xq28). The NCBI accession number of ABCD1 (Homo sapiens) is NP_000024.2 (12-MAR-2023). The Gene ID of the ABCD1 gene (Homo sapiens) in NCBI is 215, and the NCBI accession number of cDNA is NM_000033.4 (12-MAR-2023).

As used herein, the DAB channel staining intensity reflects the ABCD1 staining intensity of tumor cells, and the hematoxylin staining area reflects the number of tumor cell nuclei.

As used herein, the term "probe" refers to a single-stranded DNA or RNA sequence which is used to search for its complementary sequence in the genome of a sample. Under conditions permitting hybridization between the probe sequence and its complementary sequence, the probe is contacted with the sample to identify the target sequence. The probe is labeled with a radioactive or chemical tag for visualization of the target sequence. As used herein, the term "ABCD1-specific probe" refers to a probe that is complementary to a target sequence of ABCD1, and capable of hybridizing with and identifying the target sequence of ABCD1 under defined conditions.

As used herein, the term "primer" refers to a short single-chain DNA fragment that can be used in PCR to hybridize with sample DNA and amplify a target gene region, thereby generating many target gene fragment copies in a short time. The term "ABCD1 gene-specific primer" refers to a primer that is used for amplifying the target sequence of the ABCD1 gene.

As used herein, the term "diagnosis" refers to a process that determines the nature of diseases or conditions and differentiates them from other disorder.

### Use of ABCD1 as a marker

Disclosed herein is a novel marker, namely, ATP-binding cassette sub-family D member 1 (ABCD1), which is used for diagnosing or assisting in the diagnosis of solid pseudopapillary neoplasm of the pancreas (SPN), for differentiating or assisting in the differentiation of SPN from other non-SPN tumors, or for screening or assisting in the screening of SPN patients. In some embodiments, ABCD1 is combined with one or more markers selected from β-catenin, CD10, vimentin, and α1-antitrypsin for diagnosing or assisting in the diagnosis of SPN, for differentiating or assisting in the differentiation of SPN from other non-SPN tumors, or for screening or assisting in the screening of SPN patients.

Disclosed herein is a method for diagnosing or assisting in the diagnosis of SPN, comprising: using ABCD1 as a marker and detecting the marker for diagnosing or assisting in the diagnosis of SPN. In some embodiments, the method comprises: performing ABCD1 protein mass spectrometry analysis, qPCR detection, transcriptome sequencing, or ABCD1 immunostaining (for example, immunohistochemical staining, immunocytochemical staining, immunofluorescence staining, and multiplex immunofluorescence staining) on a clinical sample such as a tumor sample, preferably a pancreatic tumor sample, for diagnosing or assisting in the diagnosis of SPN. In some embodiments, the method comprises: performing ABCD1 immunostaining on a clinical sample and analyzing staining results, for diagnosing or assisting in the diagnosis of SPN. In some embodiments, the method comprises: performing ABCD1 immunostaining on paraffin sections or frozen sections prepared from clinical tissue samples and subjecting the staining results to manual analysis or software analysis for diagnosing or assisting in the diagnosis of SPN. In the above methods, ABCD1 can be combined with one or more markers selected from β-catenin, CD10, vimentin, and α1-antitrypsin for diagnosing or assisting in the diagnosis of SPN. In some embodiments, the ABCD1 immunostaining is performed using the antibody or the antigen-binding fragment thereof provided in the present application.

Disclosed herein is a method for differentiating or assisting in the differentiation of SPN from other non-SPN tumors, comprising: using ABCD1 as a marker and detecting the marker for differentiating or assisting in the differentiation of SPN from other non-SPN tumors. In some embodiments, the method comprises: performing ABCD1 protein mass spectrometry analysis, qPCR detection, transcriptome sequencing or ABCD1 immunostaining on a clinical sample such as a pancreatic tumor sample, for differentiating or assisting in the differentiation of SPN from other non-SPN tumors. In some embodiments, the method comprises: performing ABCD1 immunostaining on a clinical sample and analyzing staining results, for differentiating or assisting in the differentiation of SPN from other non-SPN tumors. In some embodiments, the method comprises: performing ABCD1 immunostaining on paraffin sections or frozen sections prepared from clinical tissue samples and subjecting the staining results to manual analysis or software analysis for differentiating or assisting in the differentiation of SPN from other non-SPN tumors. In some embodiments, the other non-SPN tumors comprise pancreatic acinar cell carcinoma, pancreatoblastoma, pancreatic neuroendocrine tumors (including non-functional pancreatic neuroendocrine tumor (NF-PanNET) and insulinoma) and pancreatic ductal adenocarcinoma. In some embodiments, the common immunostaining comprises immunohistochemical staining, immunocytochemical staining, immunofluorescence staining, multiplex immunofluorescence staining, etc. In the aforementioned methods, ABCD1 can be combined with one or more markers selected from β-catenin, CD10, vimentin, and α1-antitrypsin for differentiating or assisting in the differentiation of SPN. In some embodiments, the ABCD1 immunostaining is performed using the antibody or the antigen-binding fragment thereof provided in the present application.

Disclosed herein is a method for screening or assisting in the screening of SPN patients, comprising: using ABCD1 as a marker and detecting the marker for screening or assisting in the screening of SPN patients. In some embodiments, the method comprises: performing ABCD1 protein mass spectrometry detection analysis, qPCR detection, transcriptome sequencing or ABCD1 immunostaining on a clinical sample such as a pancreatic tumor sample (for example, a tumor tissue sample and a tumor cell sample) from a pancreatic tumor patient for screening or assisting in the screening of SPN patients. In some embodiments, the method comprises: performing ABCD1 protein mass spectrometry analysis, qPCR detection, transcriptome sequencing or immunostaining on a clinical sample and analyzing detection results for screening or assisting in the screening of SPN patients. In the aforementioned methods, ABCD1 can be combined with one or more markers selected from β-catenin, CD10, vimentin, and α1-antitrypsin for screening or assisting in the screening of SPN patients. In some embodiments, the common immunostaining comprises immunohistochemical staining, immunocytochemical staining, immunofluorescence staining, multiplex immunofluorescence staining, etc. In some embodiments, the ABCD1 immunostaining is performed using the antibody or the antigen-binding fragment thereof provided in the present application.

Disclosed herein is use of ABCD1 as a detection marker in any one or more of the following (a)-(c): (a) use in the development of a diagnostic reagent for diagnosing or assisting in the diagnosis of SPN; (b) use in the development of a differentiation reagent for differentiating or assisting in the differentiation of SPN from other non-SPN neoplasms; and/or (c) use in the development of a screening reagent for screening or assisting in the screening of SPN patients.

Disclosed herein is use of a substance for detecting ABCD1 in any one or more of the following (a)-(c): (a) use in the preparation of a diagnostic reagent for diagnosing or assisting in the diagnosis of SPN; (b) use in the preparation of a differentiation reagent for differentiating or assisting in the differentiation of SPN from other non-SPN tumors; and/or (c) use in the preparation of a screening reagent for screening or assisting in the screening of SPN patients.

Disclosed herein is use of a substance for detecting ABCD1 in the preparation of a kit, wherein the kit is configured for any one or more of the following (a)-(c): (a) for diagnosing or assisting in the diagnosis of SPN; (b) for differentiating or assisting in the differentiation of SPN from other non-SPN tumors; and/or (c) for screening or assisting in the screening of SPN patients.

In some embodiments, the substance for detecting ABCD1 comprises an anti-ABCD1 antibody, an ABCD1 gene-specific primer and an ABCD1 gene-specific probe. In some preferred embodiments, the substance for detecting ABCD1 comprises the antibody or the antigen-binding fragment thereof provided in the present application.

In some embodiments, the other non-SPN tumors comprise pancreatic acinar cell carcinoma, pancreatoblastoma, pancreatic neuroendocrine tumors (including non-functional pancreatic neuroendocrine tumor and insulinoma) and pancreatic ductal adenocarcinoma.

In some embodiments, the object of diagnosis or assistance in diagnosis using the diagnostic reagent is a tumor, and the object of differentiation or assistance in differentiation using the differentiation reagent is a tumor, preferably a pancreatic tumor. In some embodiments, the subject for screening or assistance in screening using the screening reagent is a tumor patient, preferably a tumor sample from a pancreatic tumor patient.

In some embodiments, the above-mentioned diagnostic reagent, differentiation reagent or screening reagent further comprises a substance for detecting one or more markers as follows: β-catenin, CD10, vimentin, and α1-antitrypsin.

### Antibody binding to ABCD1 or antigen-binding fragment thereof

Disclosed herein is an antibody binding to ABCD1 or an antigen-binding fragment thereof, wherein the complementarity-determining regions (CDRs) of the antibody or the antigen-binding fragment thereof have the following amino acid sequences: a heavy chain CDR1 selected from at least one of amino acid sequences set forth in SEQ ID NOs: 1-8; a heavy chain CDR2 selected from at least one of amino acid sequences set forth in SEQ ID NOs: 9-17; a heavy chain CDR3 selected from at least one of amino acid sequences set forth in SEQ ID NOs: 18-24; a light chain CDR1 selected from at least one of amino acid sequences set forth in SEQ ID NOs: 25-30; a light chain CDR2 selected from at least one of amino acid sequences LVS, WAS, RMS or YAS; and a light chain CDR3 selected from at least one of amino acid sequences set forth in SEQ ID NOs: 31-35.

In some embodiments, the amino acid sequences of the complementarity-determining regions of the antibody or the antigen-binding fragment thereof are selected from at least one of the following groups (1)-(10):
(1) the heavy chain CDR1, the heavy chain CDR2, the heavy chain CDR3, the light chain CDR1, the light chain CDR2, and the light chain CDR3 are amino acid sequences set forth in SEQ ID NOs: 1, 9, 18, 25, RMS, and 31, respectively;
(2) the heavy chain CDR1, the heavy chain CDR2, the heavy chain CDR3, the light chain CDR1, the light chain CDR2, and the light chain CDR3 are amino acid sequences set forth in SEQ ID NOs: 2, 10, 19, 26, YAS, and 32, respectively;
(3) the heavy chain CDR1, the heavy chain CDR2, the heavy chain CDR3, the light chain CDR1, the light chain CDR2, and the light chain CDR3 are amino acid sequences set forth in SEQ ID NOs: 3, 11, 20, 27, WAS, and 33, respectively;
(4) the heavy chain CDR1, the heavy chain CDR2, the heavy chain CDR3, the light chain CDR1, the light chain CDR2, and the light chain CDR3 are amino acid sequences set forth in SEQ ID NOs: 1, 12, 18, 25, RMS, and 31, respectively;
(5) the heavy chain CDR1, the heavy chain CDR2, the heavy chain CDR3, the light chain CDR1, the light chain CDR2, and the light chain CDR3 are amino acid sequences set forth in SEQ ID NOs: 1, 13, 18, 25, RMS, and 31, respectively;
(6) the heavy chain CDR1, the heavy chain CDR2, the heavy chain CDR3, the light chain CDR1, the light chain CDR2, and the light chain CDR3 are amino acid sequences set forth in SEQ ID NOs: 4, 14, 21, 25, RMS, and 31, respectively;
(7) the heavy chain CDR1, the heavy chain CDR2, the heavy chain CDR3, the light chain CDR1, the light chain CDR2, and the light chain CDR3 are amino acid sequences set forth in SEQ ID NOs: 5, 15, 22, 28, RMS, and 31, respectively;
(8) the heavy chain CDR1, the heavy chain CDR2, the heavy chain CDR3, the light chain CDR1, the light chain CDR2, and the light chain CDR3 are amino acid sequences set forth in SEQ ID NOs: 6, 16, 23, 29, LVS, and 34, respectively;
(9) the heavy chain CDR1, the heavy chain CDR2, the heavy chain CDR3, the light chain CDR1, the light chain CDR2, and the light chain CDR3 are amino acid sequences set forth in SEQ ID NOs: 7, 17, 24, 30, WAS, and 35, respectively; and
(10) the heavy chain CDR1, the heavy chain CDR2, the heavy chain CDR3, the light chain CDR1, the light chain CDR2, and the light chain CDR3 are amino acid sequences set forth in SEQ ID NOs: 8, 16, 23, 29, LVS, and 34, respectively.

In some embodiments, the heavy chain variable region of the antibody or the antigen-binding fragment thereof comprises an amino acid sequence with at least 75%, 80%, 85%, 90%, 95%, 98%, 99%, or 100% identity to any one of the amino acid sequences set forth in SEQ ID NOs: 36-45, and the light chain variable region of the antibody or the antigen-binding fragment thereof comprises an amino acid sequence with at least 75%, 80%, 85%, 90%, 95%, 98%, 99%, or 100% identity to any one of the amino acid sequences set forth in SEQ ID NOs: 46-55.

In some embodiments, the amino acid sequences of the heavy chain variable region and the light chain variable region of the antibody or the antigen-binding fragment thereof are selected from at least one of the following groups (1)-(10):
(1) the heavy chain variable region comprises the amino acid sequence set forth in SEQ ID NO: 36, and the light chain variable region comprises the amino acid sequence set forth in SEQ ID NO: 46;
(2) the heavy chain variable region comprises the amino acid sequence set forth in SEQ ID NO: 37, and the light chain variable region comprises the amino acid sequence set forth in SEQ ID NO: 47;
(3) the heavy chain variable region comprises the amino acid sequence set forth in SEQ ID NO: 38, and the light chain variable region comprises the amino acid sequence set forth in SEQ ID NO: 48;
(4) the heavy chain variable region comprises the amino acid sequence set forth in SEQ ID NO: 39, and the light chain variable region comprises the amino acid sequence set forth in SEQ ID NO: 49;
(5) the heavy chain variable region comprises the amino acid sequence set forth in SEQ ID NO: 40, and the light chain variable region comprises the amino acid sequence set forth in SEQ ID NO: 50;
(6) the heavy chain variable region comprises the amino acid sequence set forth in SEQ ID NO: 41, and the light chain variable region comprises the amino acid sequence set forth in SEQ ID NO: 51;
(7) the heavy chain variable region comprises the amino acid sequence set forth in SEQ ID NO: 42, and the light chain variable region comprises the amino acid sequence set forth in SEQ ID NO: 52;
(8) the heavy chain variable region comprises the amino acid sequence set forth in SEQ ID NO: 43, and the light chain variable region comprises the amino acid sequence set forth in SEQ ID NO: 53;
(9) the heavy chain variable region comprises the amino acid sequence set forth in SEQ ID NO: 44, and the light chain variable region comprises the amino acid sequence set forth in SEQ ID NO: 54; and
(10) the heavy chain variable region comprises the amino acid sequence set forth in SEQ ID NO: 45, and the light chain variable region comprises the amino acid sequence set forth in SEQ ID NO: 55.

The present application further provides a polynucleotide, wherein the polynucleotide encodes the antibody or the antigen-binding fragment thereof according to the present application.

The present application further provides a recombinant vector, wherein the recombinant vector comprises the polynucleotide according to the present application.

The present application further provides a host cell, wherein the host cell comprises the polynucleotide according to the present application and/or the recombinant vector according to the present application.

The present application further provides a pharmaceutical composition, wherein the pharmaceutical composition comprises the antibody or the antigen-binding fragment thereof according to the present application.

The present application further provides a kit, wherein the kit comprises the antibody or the antigen-binding fragment thereof according to the present application.

### ABCD1 detection reagent or detection kit

Disclosed herein is a reagent comprising a substance for detecting ABCD1, wherein the reagent is used for any one or more of the following (a)-(c): (a) the reagent is used for diagnosing or assisting in the diagnosis of SPN; (b) the reagent is used for differentiating or assisting in the differentiation of SPN from other non-SPN tumors; and/or (c) the reagent is used for screening or assisting in the screening of SPN patients.

Disclosed herein is a kit comprising a substance for detecting ABCD1, wherein the kit is configured for any one or more of the following (a)-(c): (a) for diagnosing or assisting in the diagnosis of SPN; (b) for differentiating or assisting in the differentiation of SPN from other non-SPN tumors; and/or (c) for screening or assisting in the screening of SPN patients.

In some embodiments, the substance for detecting ABCD1 comprises an anti-ABCD1 antibody, an ABCD1 gene-specific primer and an ABCD1 gene-specific probe.

In some embodiments, the reagent further comprises a substance for detecting one or more markers selected from: β-catenin, CD10, vimentin, and α1-antitrypsin.

In some embodiments, the object of diagnosis or assistance in diagnosis, differentiation or assistance in differentiation is a tumor, preferably a pancreatic tumor, and the subject for screening or assistance in screening is a tumor patient, preferably a tumor sample from a pancreatic tumor patient.

In some embodiments, any substance for detecting ABCD1 as described above is a reagent or a reagent combination for performing ABCD1 immunostaining on a clinical tissue sample (for example, the prepared paraffin section or frozen section).

For example, any substance for detecting ABCD1 as described above is the anti-ABCD1 antibody. The anti-ABCD1 antibody can be selected from recombinant anti-ABCD1 antibodies (rabbit monoclonal antibody [EPR15929], purchased from Abcam, catalog number: ab197013). In some preferred embodiments, the substance for detecting ABCD1 comprises the antibody or the antigen-binding fragment thereof provided in the present application.

Specifically, the image analysis using FIJI software according to any one of the above descriptions comprises the following steps:
(1) Import the image obtained in Step 1 into FIJI software, click process-subtract background-rolling ball radius 50.0 pixels, and check light background, and click OK; and click image-color-colour-deconvolution, select H DAB, and click OK.
(2) Select the DAB image (colour 2) obtained in Step (1), click image-type-8 bit, click analyze-calibrate, select uncalibrated OD, then click OK; click image-adjust-threshold; click analyze-Set measurements, click limit to threshold, integrated density and display label, and then click OK; click analyze-measure, thereby obtaining the integrated density, which represents the DAB staining intensity of the screenshot.
(3) Select the H image (colour 3) obtained in Step (1), click image-type-8 bit, click analyze-calibrate, select uncalibrated OD, then click OK, click image-adjust-threshold; click analyze-set measurements, check limit to threshold, area and display label, then click OK; and click analyze-measure, so as to obtain the area, which represents the hematoxylin-stained area of the screenshot.

### System for the diagnosis or differentiation of SPN

Disclosed herein is a system for diagnosing or assisting in the diagnosis of SPN, comprising a detection system and an interpretation system, wherein the detection system is used for ABCD1 immunostaining of tumor tissues or tumor cells of a subject; and the interpretation system is used for converting ABCD1 immunostaining results into interpretation results indicative SPN or non-SPN. The interpretation system can convert the immunostaining results into the interpretation result according to manual scoring or FIJI scoring. In some embodiments, the tumor tissues from the subject are paraffin sections or frozen sections of pancreatic tumor tissues. Commonly used immunostaining comprises immunohistochemical staining, immunocytochemical staining, immunofluorescence staining, multiplex immunofluorescence staining, etc. In some embodiments, the immunostaining is immunohistochemical staining or immunocytochemical staining, and results are interpreted based on expression intensity and/or distribution of ABCD1 in pancreatic tumor tissues or tumor cells. In some embodiments, the immunostaining is immunohistochemical staining or immunocytochemical staining, and the interpretation results are based on a ratio of immunohistochemical staining intensity to hematoxylin-stained area, or a ratio of immunocytochemical staining intensity to hematoxylin-stained area. In some preferred embodiments, the ABCD1 immunostaining is performed using the antibody or the antigen-binding fragment thereof provided in the present application.

In some embodiments, the interpretation system is specifically a system for executing the following operations:
Following ABCD1 immunohistochemical staining, with staining defined as tan/brown staining of the cytoplasm and cell membrane, and differentiation is performed according to staining intensity and proportion as follows: no staining or staining proportion < 5%: classified as negative (-); staining proportion ≥ 5% and < 50%, with weak and incomplete staining: classified as weakly positive (+); staining proportion ≥ 50% and < 80%, with moderate or focal complete membrane staining: classified as moderately positive (++); staining proportion ≥ 80%, with strong and complete staining: classified as strongly positive (+++);

A score of - or + is interpreted as non-SPN, while a score of ++ or +++ is interpreted as SPN.

The interpretation system is specifically a system for executing the following operations:
1. After ABCD1 immunohistochemical staining, the sections are scanned into digital slides using the NanoZoomer S360 digital slide scanner under bright-field, ×40 magnification, single-layer mode, and auto-focus. The digital slides are imported into the NDP.view2 viewing software. Five random tumor regions are selected under the 20× objective lens and exported (format: JPEG; resolution: 300 dpi);
2. Five screenshots obtained from Step 1 for each section are subjected to image analysis using FIJI software. The scoring result = DAB channel staining intensity (reflecting ABCD1 staining intensity in tumor cells)/hematoxylin-stained area (reflecting the number of tumor cell nuclei). Statistical analysis is performed using the mean value of the scoring results from the five screenshots. Using 0.1089 as the threshold, a scoring result ≤ the threshold is judged as non-SPN, and a scoring result > the threshold is judged as SPN.

Disclosed herein is a system for differentiating or assisting in the differentiation of SPN from other tumors, comprising a detection system and an interpretation system, wherein the detection system is used for ABCD1 immunostaining of tumor tissues or tumor cells in a subject; and the interpretation system is used for converting the ABCD1 immunostaining results into interpretation results indicative of SPN or other non-SPN tumors. The interpretation system can convert the immunostaining results into the interpretation result according to manual scoring or FIJI scoring, etc. In some embodiments, the system is used for differentiating or assisting in the differentiation of a tumor, preferably a pancreatic tumor. In some embodiments, the other non-SPN tumors comprise pancreatic acinar cell carcinoma, pancreatoblastoma, pancreatic neuroendocrine tumors (including non-functional pancreatic neuroendocrine tumors (NF-PanNET)) and pancreatic ductal adenocarcinoma. In some embodiments, the tumor tissues from the subject are paraffin sections or frozen sections of pancreatic tumor tissues. In some embodiments, the immunostaining is immunohistochemical staining or immunocytochemical staining, and the interpretation results are based on expression intensity and/or distribution of ABCD1 in tumor tissues or cells. In some embodiments, the immunostaining is immunohistochemical staining or immunocytochemical staining, and the interpretation results are based on a ratio of immunohistochemical staining intensity to hematoxylin-stained area, or a ratio of immunocytochemical staining intensity to hematoxylin-stained area. In some embodiments, the interpretation results are based on a ratio of DAB channel staining intensity to hematoxylin-stained area. In some preferred embodiments, the ABCD1 immunostaining is performed using the antibody or the antigen-binding fragment thereof provided in the present application.

In some embodiments, the interpretation system is specifically a system for executing the following operations:
Following ABCD1 immunohistochemical staining, labeling is defined as tan/brown staining of the cytoplasm and cell membrane, and differentiation is performed according to staining intensity and proportion as follows: no staining or staining proportion < 5%: classified as negative (-); staining proportion ≥ 5% and < 50%, with weak and incomplete staining: classified as weakly positive (+); staining proportion ≥ 50% and < 80%, with moderate or focal complete membrane staining: classified as moderately positive (++); staining proportion ≥ 80%, with strong and complete staining: classified as strongly positive (+++);

A score of - or + is interpreted as other non-SPN tumors, while a score of ++ or +++ is interpreted as SPN.

The interpretation system is specifically a system for executing the following operations:
1. After ABCD1 immunohistochemical staining, the sections are scanned into digital slides using the NanoZoomer S360 digital slide scanner under brightfield, ×40 magnification, single-layer mode, and auto-focus. The digital slides are imported into the NDP.view2 viewing software. Five random tumor regions are selected under the 20× objective lens and exported (format: JPEG; resolution: 300 dpi);
2. Five screenshots obtained from Step 1 for each section are analyzed using the FIJI software. Score = DAB channel staining intensity (reflecting ABCD1 staining intensity in tumor cells)/hematoxylin-stained area (reflecting the number of tumor cell nuclei). Statistical analysis is performed using the mean score of the five screenshots. With 0.1089 as the threshold, a score ≤ threshold is diagnosed as other non-SPN tumors, and a score > threshold is diagnosed as SPN.

### Example

Exemplary examples of the present application are described with reference to drawings, which include various details of the examples of the present application to facilitate understanding. It should be understood that these are exemplary only and in no way intended to limit the protection scope of the present application. The protection scope of the present application is defined solely by claims. Therefore, persons of ordinary skill in the art should recognize that various changes and modifications can be made to the embodiments described herein without departing from the scope of the present application. Also, for clarity and conciseness, descriptions of well-known functions and structures are omitted in the following description.

Unless otherwise specified, the experimental methods in the following examples are conventional methods, performed according to the techniques or conditions described in the literature in the art or in accordance with the product instructions. Unless otherwise specified, the materials, reagents and other items used in the following examples are all commercially available.

### Example 1: Identification of ABCD1 as a diagnostic marker

### I. Increased peroxisome number in pancreatic tumor tissues from SPN patients

Peroxisomes are single-membrane-bound intracellular organelles primarily responsible for biochemical reactions such as lipid metabolism, redox reactions and metabolism of reactive oxygen species metabolism. The size, morphology and number of the peroxisomes vary among different cell types and species. The formation and maintenance of the peroxisome require the synergistic action of a series of peroxisomal membrane proteins (including channel proteins) and enzymatic proteins. Currently known membrane proteins and channel proteins of peroxisomes include but are not limited to: membrane proteins (PEX1, PEX3, PEX5, PEX7, PEX10, PEX11, PEX13, PEX14, PEX16, PEX19, PEX22, and PEX26), channel proteins (ABCD1, ABCD2, ABCD3, and ABCD4), etc. Currently known enzymatic proteins of the peroxisomes include but are not limited to: fatty acyl-CoA oxidase (ACOX), catalase (CAT), acyl-CoA oxidase (ACOX), α-oxidation, β-oxidation, D-3-hydroxyacyl-CoA dehydrogenase (HSD17B4), and D-2,4-dienoyl-CoA reductase (DECR).

The transcriptome data of pancreatic tumor tissues and adjacent normal pancreatic tissues from SPN patients as well as the transcriptome data of pancreatic tumor tissues from SPN patients and NF-PanNET patients are compared. Differentially expressed genes were further statistically analyzed using software such as DESeq2/edgeR and Limma/DEP, and functionally annotated using GO and KEGG. The analysis revealed that genes related to peroxisomal membrane proteins and channel proteins (PEX1, PEX3, PEX10, PEX11B, PEX13, PEX26, ABCD1, ABCD3, and ABCD4) and genes related to enzymatic proteins (ACOX1 and ACOX3) were upregulated in pancreatic tumor tissues from SPN patients, suggesting that the number or function of peroxisomes may be abnormal in pancreatic tumor tissues of SPN patients.

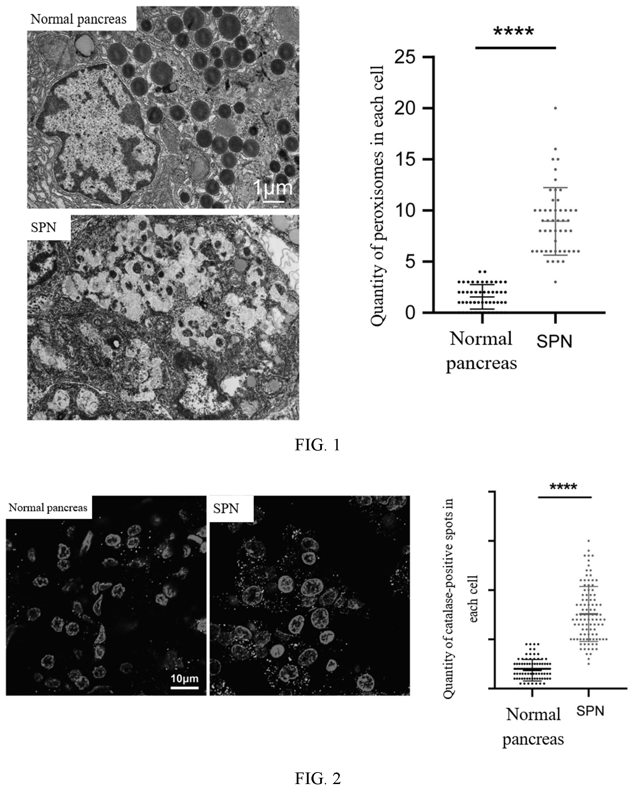

Based on the above results, the inventors collected pancreatic tumor tissues and adjacent normal pancreatic tissues from SPN patients, and performed transmission electron microscopy and catalase immunofluorescence staining, respectively (catalase is an enzyme in peroxisomes that can serve as an indicator of peroxisome abundance). Representative transmission electron microscopy images and statistical results are shown in FIG. 1. In FIG. 1, SPN indicates pancreatic tumor tissues from SPN patients, and normal pancreas indicates adjacent normal pancreatic tissues from SPN patients. The vertical axis of the right panel represents the average number of peroxisomes per cell. Representative immunofluorescence staining images and statistical results are shown in FIG. 2. In FIG. 2, SPN indicates pancreatic tumor tissues from SPN patients, and normal pancreas indicates adjacent normal pancreatic tissues from SPN patients. The vertical axis of the right panel represents the number of catalase-positive dots per cell. Compared with adjacent normal pancreatic tissues, the number of peroxisomes in pancreatic tumors from SPN patients increases.

### II. Increased abundance of ABCD1 in pancreatic tumor tissues from SPN patients

ABCD1, full name ATP-binding cassette sub-family D member 1. ABCD1 is a member of the ABC transporter superfamily. ABC transporters are classified into seven distinct subfamilies (ABC1, MDR/TAP, MRP, ALD, OABP, GCN20 and White). ABCD1 is a member of the ALD subfamily and is involved in the peroxisomal transport of long-chain fatty acids and/or fatty acyl-CoA into the peroxisome. All known peroxisomal ABC transporters are half-transporters that require association with another half-transporter molecule to form functional homodimeric or heterodimeric transporter complexes. The ABCD1 gene is mapped to the long arm of the human X chromosome (Xq28). The NCBI accession number of ABCD1 (Homo sapiens) is NP_000024.2 (12-MAR-2023). The Gene ID of the ABCD1 gene (Homo sapiens) in NCBI is 215, and the NCBI accession number of cDNA is NM_000033.4 (12-MAR-2023).

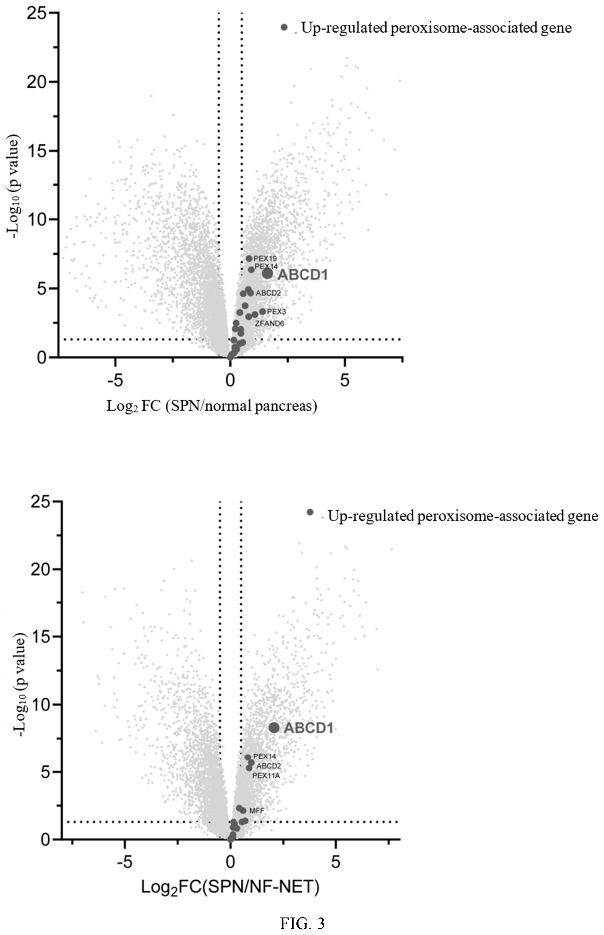

By analyzing transcriptome data from pancreatic tumor tissues and adjacent normal pancreatic tissues of SPN patients, as well as transcriptome data from pancreatic tumor tissues in SPN patients and NF-PanNET patients, it is revealed that ABCD1 is upregulated in pancreatic tumor tissues from SPN patients, with Log₂ FC = 1.622 compared with adjacent normal pancreatic tissues and Log₂ FC = 2.057 compared with pancreatic tumor tissues of NF-PanNET patients. Volcano plots illustrating differentially expressed genes are presented in FIG. 3, including comparisons between pancreatic tumor tissues and adjacent normal pancreatic tissues in SPN patients, as well as between pancreatic tumor tissues of SPN patients and NF-PanNET patients. In FIG. 3, SPN denotes pancreatic tumor tissue from SPN patients, normal pancreas denotes adjacent normal pancreatic tissue from SPN patients, and NF-PanNET denotes pancreatic tumor tissue from NF-PanNET patients.

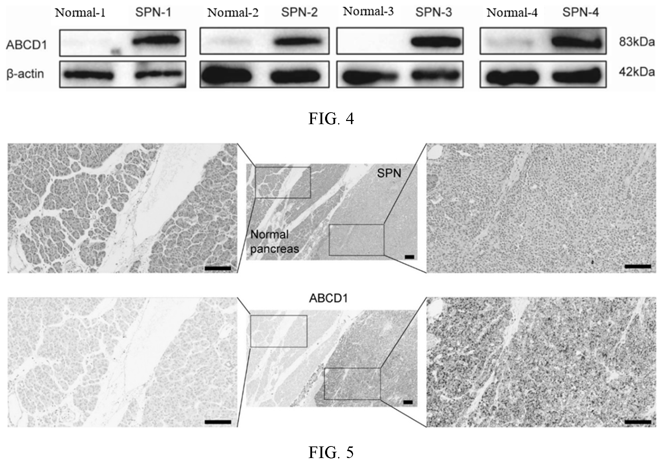

Pancreatic tumor tissues and adjacent normal pancreatic tissues from SPN patients were harvested for western blotting analysis (ABCD1 antibody is used as primary antibody; detailed antibody information is provided in Example 3). The results are shown in FIG. 4. In FIG. 4, -1, -2, -3, and -4 represent four independent SPN patients, while SPN indicates pancreatic tumor tissues from SPN patients, and normal indicates adjacent normal pancreatic tissues from SPN patients.

Paraffin sections of pancreatic tissues from SPN patients (serial sections) were subjected to HE staining and IHC staining for ABCD1, respectively (the protocol for ABCD1 IHC staining is described in Example 3). Representative images are shown in FIG. 5 (upper panel: HE staining; lower panel: ABCD1 IHC staining). In FIG. 5, SPN indicates pancreatic tumor tissue from SPN patients, and normal pancreas indicates adjacent normal pancreatic tissue from SPN patients.

### Example 2: Validation of ABCD1 as a diagnostic marker

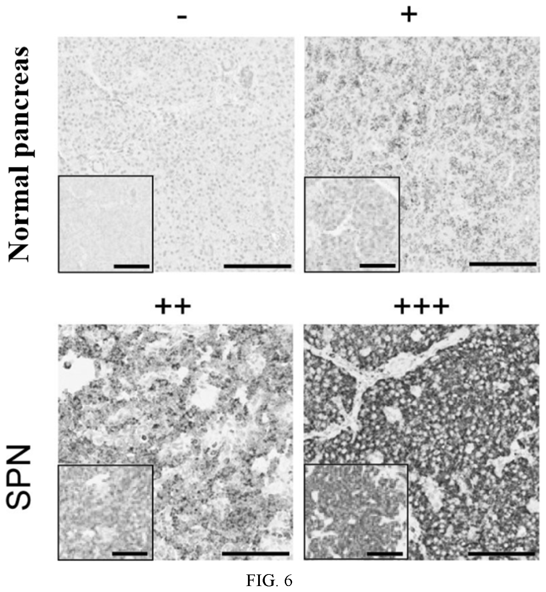

Paraffin sections of pancreatic tumor tissues and adjacent normal pancreatic tissues from 50 patients with pathologically confirmed SPN who underwent surgical resection at Peking Union Medical College Hospital between January 2016 and April 2022 were collected. Immunohistochemical staining for ABCD1 was performed, followed by scoring (the staining and scoring methods are the same as those described in Example 3). Representative images of each staining score is shown in FIG. 6. In FIG. 6, SPN indicates pancreatic tumor tissue from SPN patients, and normal pancreas indicates adjacent normal pancreatic tissue from SPN patients.

Paraffin-embedded tissue samples of extrapancreatic SPN and corresponding clinicopathological data were collected from patients who underwent surgical resection and were pathologically confirmed at Peking Union Medical College Hospital between January 2016 and April 2022. A total of 16 patients with metastatic SPN were enrolled, with relevant information as follows: one patient developed two successive liver metastases, and another patient presented with simultaneous retroperitoneal mass and liver metastasis, resulting in a total of 18 metastatic lesions (8 liver metastases, 2 retroperitoneal masses, 4 intra-abdominal masses, 3 lymph node metastastases, and 1 ovarian metastasis). Paraffin sections of metastatic lesion were subjected to IHC staining for ABCD1, followed by scoring (the staining and scoring methods are the same as those described in Example 3). All metastatic lesions were strongly positive (+++).

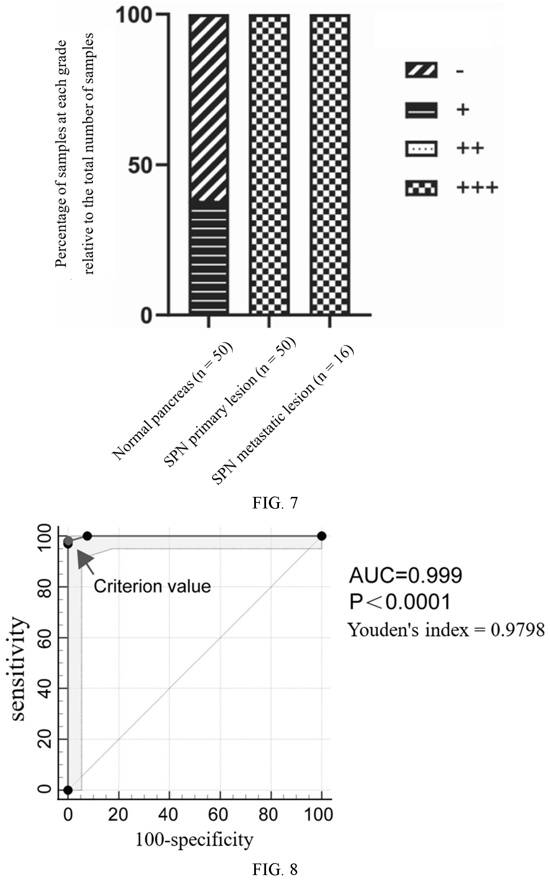

The scoring results of pancreatic tumor tissues and adjacent normal pancreatic tissues from 50 SPN patients as described above, as well as the scoring results of metastatic lesion tumor tissues from 16 SPN patients as described above, are shown in FIG. 7. In FIG. 7, SPN primary lesions represent primary pancreatic tumor tissues from SPN patients (n = 50); normal pancreas indicates adjacent normal pancreatic tissues from SPN patients (n = 50); SPN metastatic lesions indicate metastatic tumor tissues from SPN patients (n = 18). The vertical axis represents the percentage of samples at each score relative to the total number of samples.

The results show that ABCD1 expression is upregulated in both primary and metastatic tumor lesions from SPN patients.

### Example 3: Performance evaluation of ABCD1 as a diagnostic marker

Paraffin-embedded tissue samples and corresponding clinicopathological data were obtained from SPN patients and NF-PanNET patients who underwent surgical resection and were pathologically confirmed at Peking Union Medical College Hospital between December 2016 and June 2022. A total of 99 SPN patients were enrolled, with relevant information as follows: 20 patients were male and 79 patients were female, with a median age of 31 years; and a total of 91 NF-PanNET patients were enrolled, including 39 males and 52 females, with a median age of 52 years.

### I. ABCD1 IHC staining of pancreatic tumor tissues

1. Paraffin-embedded tissue samples were obtained, and 4 µm-thick paraffin -embedded tissue sections were prepared.
2. The paraffin-embedded tissue sections obtained in Step 1 were subjected to baking, dewaxing and hydration in sequence.
   Baking: sections were placed in an oven at 60°C for at least 60 min.
   Dewaxing: sections were immersed in xylene for 20 min, xylene for 20 min.
   Hydration: sections were treated with 100% ethanol for 10 min, 100% ethanol for 10 min, 95% ethanol for 5 min, 90% ethanol for 5 min, 85% ethanol for 5 min, 70% ethanol for 5 min, followed by rinsing with PBS buffer.
3. After completing Step 2, the paraffin-embedded tissue sections were subjected to antigen retrieval, followed by rinsing with PBS buffer.
   Antigen retrieval: sodium citrate antigen retrieval buffer was added to a pressure cooker, and the rinsed paraffin sections were fully immersed in it. The pressure cooker was placed in a microwave and heated until boiling. The lid was opened to check for bubbles (the presence of bubbles indicated that the sodium citrate antigen retrieval buffer had boiled). After closing the lid, heating was continued for 5 min. The lid was then opened, and the sections were cooled naturally at room temperature.
4. The paraffin sections processed in Step 3 were taken to block endogenous peroxidase activity, followed by rinsing with PBS buffer.
   Blocking of endogenous peroxidase: paraffin sections were completely immersed in 3% hydrogen peroxide solution and incubated in the dark at room temperature for 30 min.
5. The paraffin sections processed in Step 4 were blocked with BSA blocking solution at room temperature for 60 min.
   BSA blocking solution: containing 5% BSA in 0.1% PBST buffer.
6. The paraffin sections processed in Step 5 were incubated with a primary antibody working solution for 2 h, followed by rinsing with PBS buffer.
   Primary antibody working solution: ABCD1 antibody was diluted 100-fold with PBS buffer. ABCD1 antibody: Recombinant anti-ABCD1/ALD antibody, rabbit monoclonal antibody [EPR15929] against ABCD1/ALD, Abcam, Cat. No. ab197013.
7. The paraffin sections processed in Step 6 were incubated in the secondary antibody working solution for 1 h. A biotin substrate was then added dropwise, followed by incubation for 30 min at room temperature. The sections were rinsed with PBS buffer.
   Secondary antibody working solution: HRP-conjugated secondary antibody diluted 1000-fold with PBS buffer. HRP-conjugated secondary antibody: Goat anti-Rabbit IgG H&L (HRP), Abcam, Cat. No. ab6721.
8. 1×DAB developing solution was added dropwise to the paraffin sections processed in Step 7. The color development was observed under a microscope (the staining reaction was terminated promptly with tap water), and the sections were rinsed with tap water.
9. The paraffin sections processed in Step 8 were immersed in a hematoxylin staining solution for 10-20 s, followed by rinsing with tap water and soaking in PBS buffer (pH 7.2-7.4) for 10 min.
10. The paraffin sections processed in Step 9 were subjected to tissue dehydration, paraffin clearing and mounting in sequence.
   Tissue dehydration: soak in 70% ethanol for 5 min, 85% ethanol for 5 min, 90% ethanol for 5 min, 95% ethanol for 5 min, 100% ethanol for 10 min, and 100% ethanol for 10 min.
   Paraffin clearing: soak in xylene for 20 min, xylene for 20 min.

### II. Manual scoring

A method for manual scoring of pathological tissue sections prepared in Step 1 (positive staining appeared as brown in the cytoplasm and cell membrane; and sections were graded according to staining intensity and proportion): no staining or staining proportion < 5% was classified as negative (-); staining proportion ≥ 5% and < 50% with weak and incomplete staining was classified as weakly positive (+); staining proportion ≥ 50% and < 80% with moderate or locally complete cell membrane staining was classified as moderately positive (++); and staining proportion ≥ 80% with strong and complete staining was classified as strongly positive (+++). All sections were independently evaluated by two qualified pathologists, and a consensus was reached for the final score.

Manual scoring results showed that among 99 SPN patients, 96 patients were rated as 3+ (i.e., +++), 2 patients were rated as 2+ (i.e., ++), and 1 patient was rated as 1+ (i.e., +). Among 91 patients with NF-PanNET, 7 patients were rated as 1+ (i.e., +), and 84 patients were rated as negative (-).

Patients scored as - or + were classified as non-SPN patients, while those scored as ++ or +++ were classified as SPN patients. The ROC curve is as shown in FIG. 8. The sensitivity was 97.98%, the specificity was 100%, and the Youden's index was 0.9798. It can be seen that ABCD1 IHC staining combined with manual scoring can effectively differentiate SPN patients from those with NF-PanNET.

### III. FIJI scoring

A method for FIJI scoring of pathological tissue sections prepared in Step 1 is as follows:
1. The pathological tissue sections prepared in Step 1 were scanned into digital slides using a NanoZoomer S360 digital slide scanner (C13220-01, Hamamatsu) under bright-field, ×40 magnification, single-layer, and auto-focus mode. The digital slides were imported into NDP.view2 viewing software. Five tumor regions were randomly selected at ×20 magnification and exported. (Format: JPEG; resolution: 300 dpi).
2. Five screenshots obtained from each section in Step 1 were subjected to image analysis using FIJI software.
   (1) Import the image obtained in Step 1 into FIJI software, background subtraction was performed with a rolling ball radius 50.0 pixels, and check light background option, click OK; and click image-color-colour-deconvolution, select H DAB, and then click OK.
   (2) Select the DAB image (colour 2) obtained in Step (1), click image-type-8 bit, click analyze-calibrate, select uncalibrated OD, and then click OK; click image-adjust-threshold; clicking analyze-set measurements, click limit to threshold, integrated density and display label, and then click OK; and click analyze-measure, so as to obtain the integrated density, i.e., the DAB staining intensity of the screenshot.
   (3) Select the H image (colour 3) obtained in step (1); click image-type-8 bit; click analyze-calibrate, select Uncalibrated OD and click OK; click image-adjust-threshold; click analyze-set measurements; check limit to threshold, area and display label, then click OK; and click analyze-measure to obtain the area, i.e., the hematoxylin-stained area of the screenshot (the hematoxylin-stained area was used to reflect the number of cells).$Score = DAB staining intensity / Hematoxylin - stained area$

Statistical analysis was performed using the mean score calculated from five screenshots of each pathological tissue section.

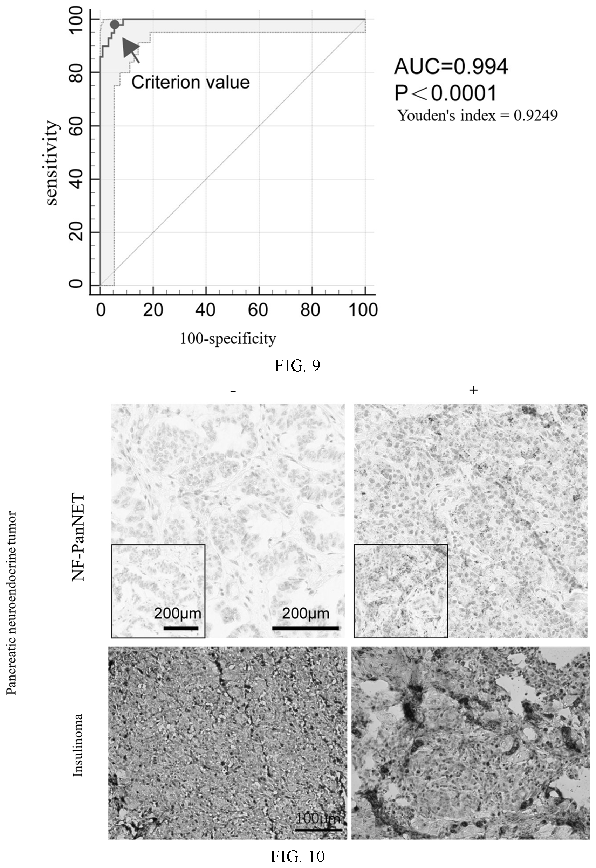

0.1089 was used as a threshold. The scores ≤ the threshold were classified as non-SPN, and scores > the threshold were classified as SPN. The ROC curve is shown in FIG. 9. The sensitivity was 97.98%, the specificity was 94.51%, and the Youden's index was 0.9249. It can be seen that ABCD1 IHC staining combined with FIJI scoring can effectively differentiate SPN from NF-PanNET patients.

### Example 4: ABCD1 abundance in other various pancreatic tumor patients

Paraffin-embedded tissue samples and corresponding clinicopathological data were obtained from patients with pancreatic neuroendocrine tumors (including NF-PanNET and insulinoma) who underwent surgical resection and were pathologically confirmed at Peking Union Medical College Hospital between August 2011 and November 2021. A total of 91 NF-PanNET patients were enrolled, including 39 males and 52 females, with a median age of 52 years. 10 patients with insulinoma were enrolled, including 8 females and 2 males, with a median age of 56 years. Paraffin-embedded tissue samples and corresponding clinicopathological data were obtained from patients with pancreatic acinar cell carcinoma who underwent surgical resection and were pathologically confirmed at Peking Union Medical College Hospital between August 2011 and November 2021. A total of 9 patients with pancreatic acinar cell carcinoma were enrolled, including 4 females and 5 males, with a median age of 58 years. Paraffin-embedded tissue samples and corresponding clinicopathological data were obtained from patients with pancreatoblastoma who underwent surgical resection and were pathologically confirmed at Peking Union Medical College Hospital between August 2011 and November 2021. A total of 3 patients with pancreatoblastoma were enrolled, including 1 female and 2 males, with a median age of 32 years. Paraffin-embedded tissue samples and corresponding clinicopathological data were obtained from patients with pancreatic ductal adenocarcinoma who underwent surgical resection and were pathologically confirmed at Peking Union Medical College Hospital between August 2011 and November 2021. A total of 10 patients with pancreatic ductal adenocarcinoma were enrolled, including 1 female and 9 males, with a median age of 61 years.

### I. ABCD1 IHC staining of pancreatic tumor tissues

The same procedures as in Step 1 of Example 3 were performed.

### II. Manual scoring

The same procedures as in Step 2 of Example 3 were performed.

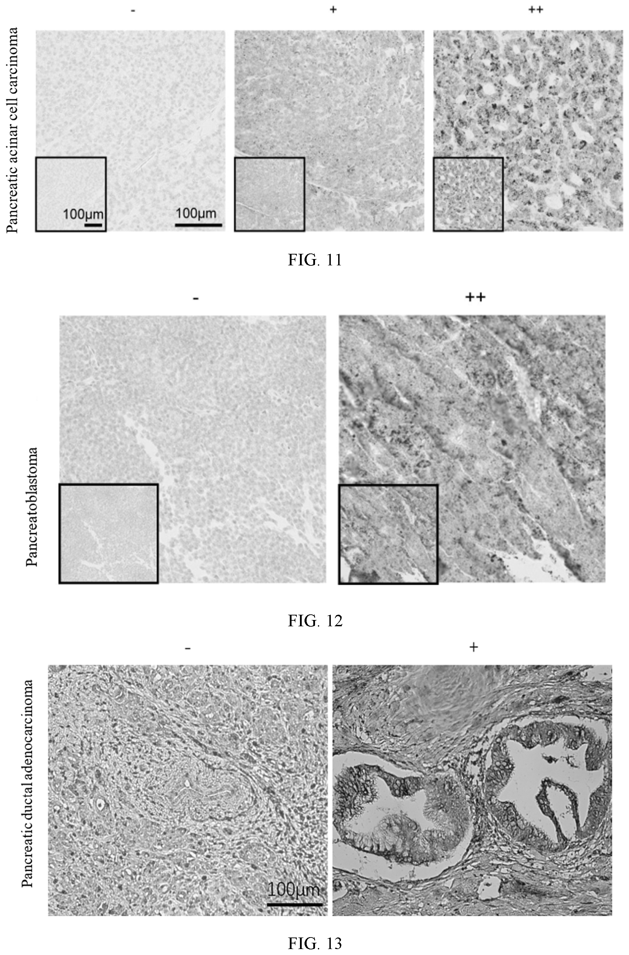

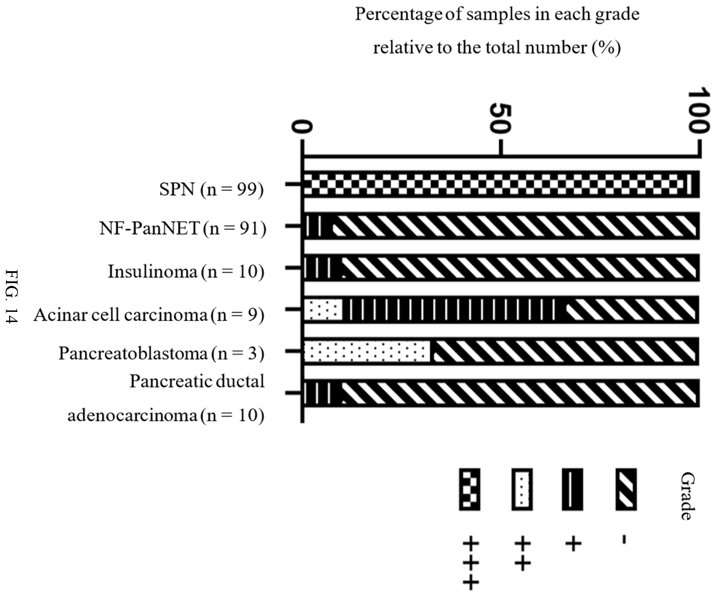

Representative IHC staining images of pancreatic tumor tissues from patients with pancreatic neuroendocrine tumors (including NF-PanNET and insulinoma) are shown in FIG. 10. Representative IHC staining images of pancreatic tumor tissues from patients with pancreatic acinar cell carcinoma are shown in FIG. 11. Representative IHC staining images of pancreatic tumor tissues from pancreatoblastoma are shown in FIG. 12. Representative IHC staining images of pancreatic tumor tissues from patients with pancreatic ductal adenocarcinoma are shown in FIG. 13. The scoring results of 99 SPN patients in Example 3, as well as 91 NF-PanNET patients in Example 4, 10 patients with insulinoma, 9 patients with acinar cell carcinoma, 3 patients with pancreatoblastoma and 10 patients with pancreatic ductal adenocarcinoma in Example 4 are shown in FIG. 14. In FIG. 14, the vertical axis represents the percentage of samples corresponding to each score in the group relative to the total number of samples in the group. Among 99 SPN patients, 96 patients were rated as +++, 2 patients were rated as ++, and 1 patient was rated as +. Among 91 NF-PanNET patients, 7 patients were rated as +, and 84 patients were rated as -. Among 10 patients with insulinoma, 9 patients were rated as -, and 1 patient was rated as +. Among 9 patients with pancreatic acinar cell carcinoma, 3 patients were rated as -, 5 patients were rated as +, and 1 patient was rated as ++. Among 3 patients with pancreatoblastoma, 2 patients were rated as -, and 1 patient was rated as ++. Among 10 patients with pancreatic ductal adenocarcinoma, 9 patients were rated as -, and 1 patient was rated as +. The results demonstrate that ABCD1 IHC staining combined with manual scoring can effectively differentiate SPN from NF-PanNET, insulinoma, pancreatic acinar cell carcinoma, pancreatoblastoma and pancreatic ductal adenocarcinoma.

### Example 5: Preparation and activity assay of anti-ABCD1 antibody

### 1. Animal immunization

Balb/c mice were immunized with 50 µg/mouse using immunogens consisting of prokaryotically expressed and purified antigen protein ABCD1 (recombinantly expressed in an *E. coli* expression system, with an amino acid sequence set forth in SEQ ID NO: 56) and synthetic polypeptide (chemically synthesized, with an amino acid sequence set forth in SEQ ID NO: 57) as immunogens. The mice received a primary immunization followed by a booster immunization half a month later, with additional booster immunizations administered at 2-week intervals. Negative serum samples were collected from mice 3 days before immunization. On day 6 after each immunization, 50 µL of blood was collected via tail clipping. Negative serum and immune serum were diluted serially (1:0.2K, 1:0.4K, 1:0.8K, 1:1.6K, 1:3.2K, 1:6.4K and 1:12.8K), and serum titer detection was performed by Cell-ELISA using ABCD1 antigen protein as the target. When the titer met the requirement and the anti-human ABCD1 antibody was detected at a dilution higher than 1:12.8K, the spleen and lymph nodes of the mice were harvested.

### 2. Cell fusion

(1) Healthy SP2/0 cells were gently pipetted off the culture flask wall and transferred into a 50 mL centrifuge tube.
(2) Blood was collected from mice by enucleation, followed by cervical dislocation. The mice were then soaked in 75% ethanol for 5 min.
(3) A small amount of serum-free IMDM was added into a petri dish, and a cell strainer and a syringe plunger were placed into the dish. The mouse spleen was dissected using scissors and forceps and placed on the cell strainer. The spleen was gently and thoroughly ground using the syringe plunger. The dispersed cells were collected into a centrifuge tube containing SP2/0 cells and centrifuged at 1500 rad/min for 5 min.
(4) The mouse thymus was harvested using scissors and forceps and ground into a single-cell suspension. The ground thymocytes were transferred into a 15 mL centrifuge tube, supplemented with 2 mL HAT medium and 1 mL HT medium, and stored in an incubator for later use.
(5) The supernatant of the centrifuged cells was discarded. The cell pellet was gently and carefully resuspended in serum-free IMDM and centrifuged at 1500 rad/min for 5 min.
(6) The supernatant of the centrifuged cells was discarded to the greatest extent. The bottom of the centrifuge tube was tapped to fully suspend the cells. The centrifuge tube was placed in 37°C warm water, and 1 mL of PEG was slowly added over approximately 1 min. After the addition was completed, the mixture was allowed to stand in warm water for 1 min. Then, 2 mL of serum-free IMDM was slowly added in 2 min, followed by slowly adding 8 mL of serum-free IMDM in 2 min. The mixture was centrifuged for 5 min at 1000 rad/min.
(7) The supernatant was discarded, 10 ml of serum was added, and the cells were carefully resuspended by pipetting and then combined with the previously prepared thymocytes. Subsequently, a sterilized semi-solid medium was added to bring a final volume of 50 mL and then mixed thoroughly. The mixture was then evenly dispensed into 30 cell culture dishes. The cell culture dishes were put in a humid chamber and then incubated in an incubator.

### 3. Preliminary screening of positive clones via ELISA

(1) The "2023-601 project-ABCD1 protein" (recombinantly expressed in *E. coli* prokaryotic system, with an amino acid sequence set forth in SEQ ID NO: 56) was diluted with coating buffer to a final concentration of 2 µg/mL, added at 100 µL per well, incubated overnight at 4°C, and then washed three times with washing buffer.
(2) The above washed protein was blocked with a blocking solution (2% skimmed milk powder) at 200 µL/well, incubated at 37°C for 2 h, and then washed three times with washing buffer.
(3) The primary antibody (cell culture supernatant), negative control (SP2/0 culture supernatant), blank control (PBS) and positive control (positive serum diluted 1000-fold with PBS) were added, each at 100 µL/well. Then, the wells were incubated at 37°C for 1 h and then washed three times with washing buffer.
(4) The secondary antibody was diluted 20,000-fold in PBS, 100 µL/well. Then, the wells were incubated at 37°C for 1 h. The plate was taken out and then washed three times with washing buffer.
(5) A developing solution was added, with 100 µL/well. The developing time was about 10 min.
(6) 50 µL of stop solution was added in each well.
(7) The absorbance was measured at dual wavelengths (450 nm and 630 nm), and the data were recorded and stored. The absorbance was read at 450 nm using a microplate reader, and the data was analyzed. Cell lines with supernatant OD450 > 0.2 were selected as primary candidate positive cell lines. The culture supernatant of the positive cell lines was aspirated and discarded, and fresh HAT complete medium was added.

### 4. Construction and in vitro recombinant expression of antibody plasmids

The antibodies screened in Step 3 were subjected to hybridoma cell sequencing. The antibody fragments obtained by sequencing were subjected to gene synthesis and cloned into the IgG framework of mouse IgG1, IgG2a or IgG2b. Subsequently, the antibody fragments were inserted into the PTT5 vector (Global Biotechnology Co., Ltd., Anhui) using a molecular cloning technique to construct a mammalian cell expression plasmid. The plasmid was transfected into the CHO host cell line (Mab Select Sure LX, Cytiva, 17547403) via lipofection. The culture supernatant was harvested and purified by Protein A affinity chromatography (expression system: CHO-S; column: Mab Select Sure LX, Cytiva, 17547403). Finally, purified recombinant antibody was obtained. The amino acid sequences of the CDRs and variable regions are shown in Table 1 below.

**Table 1 CDR sequence of antibody**

| Antibody Name | Sequences or Sequence Numbers of HCDR1, HCDR2, HCDR3, LCDR1, LCDR2 and LCDR3 | Sequence Numbers of V_{H} and V_{L} |
|---|---|---|
| 07PC03 | SEQ ID NOs: 1, 9, 18, 25, RMS, and 31 | SEQ ID NOs: 36 and 46 |
| 07PC08 | SEQ ID NOs: 2, 10, 19, 26, YAS, and 32 | SEQ ID NOs: 37 and 47 |
| 07PC09 | SEQ ID NOs: 3, 11, 20, 27, WAS, and 33 | SEQ ID NOs: 38 and 48 |
| 07PC17 | SEQ ID NOs: 1, 12, 18, 25, RMS, and 31 | SEQ ID NOs: 39 and 49 |
| 07PC27 | SEQ ID NOs: 1, 13, 18, 25, RMS, and 31 | SEQ ID NOs: 40 and 50 |
| 07PC28 | SEQ ID NOs: 4, 14, 21, 25, RMS, and 31 | SEQ ID NOs: 41 and 51 |
| 07PC44 | SEQ ID NOs: 5, 15, 22, 28, RMS, and 31 | SEQ ID NOs: 42 and 52 |
| 07TE02 | SEQ ID NOs: 6, 16, 23, 29, LVS, and 34 | SEQ ID NOs: 43 and 53 |
| 07TE04 | SEQ ID NOs: 7, 17, 24, 30, WAS, and 35 | SEQ ID NOs: 44 and 54 |
| 07TE08 | SEQ ID NOs: 8, 16, 23, 29, LVS, and 34 | SEQ ID NOs: 45 and 55 |

### 5. The binding activity of purified antibody

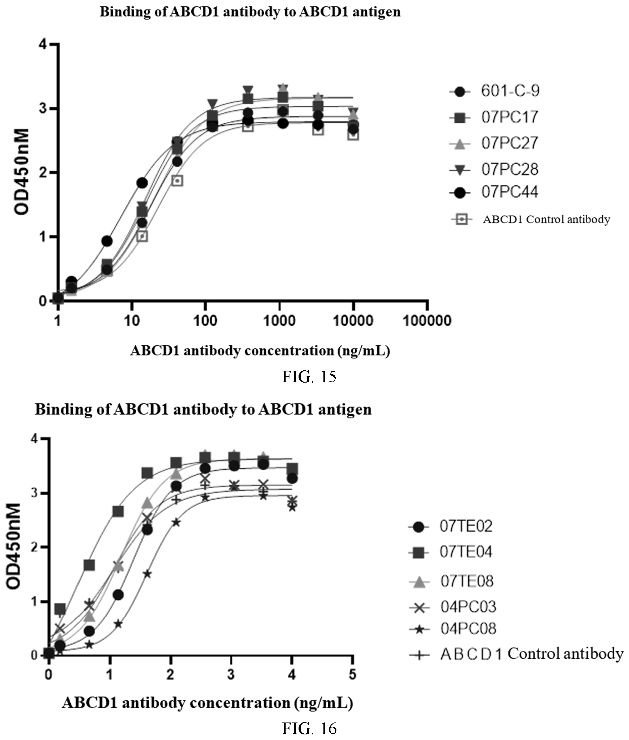

The binding activity of the antibodies listed in Table 1 was detected by ELISA. Goat antimouse secondary antibody conjugated with HRP (Abcam; Ab205719) was used as the secondary antibody. The detection protocol was identical to that of the ELISA method in Step 3. The results are shown in FIG. 15 and FIG. 16. The results showed that all 10 antibodies disclosed in Table 1 exhibited excellent binding activity to the ABCD1 protein.

### Example 6: Immunohistochemical staining of pancreatic tumor tissues using an anti-ABCD1 antibody

The antibody 07TE02 prepared in Example 5 was used for immunohistochemical staining of pancreatic tumor tissues. The clinical sample information and immunohistochemical staining method were the same as those described in Example 3.

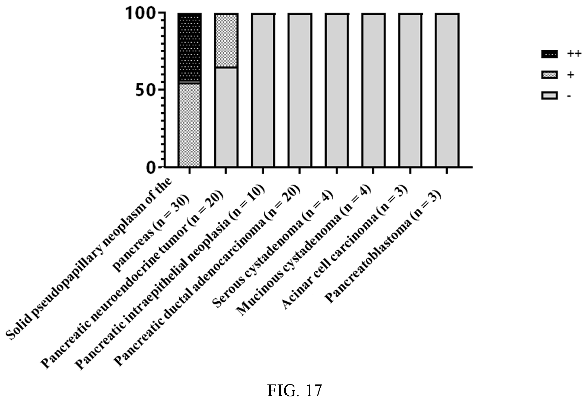

The scoring results for 30 cases of solid pseudopapillary neoplasm of the pancreas, 20 cases of pancreatic neuroendocrine tumor, 10 cases of pancreatic intraepithelial neoplasia, 20 cases of pancreatic ductal adenocarcinoma, 4 cases of serous cystadenoma, 4 cases of mucinous cystadenoma, 3 cases of acinar cell carcinoma, and 3 cases of pancreatoblastoma are shown in FIG. 17.

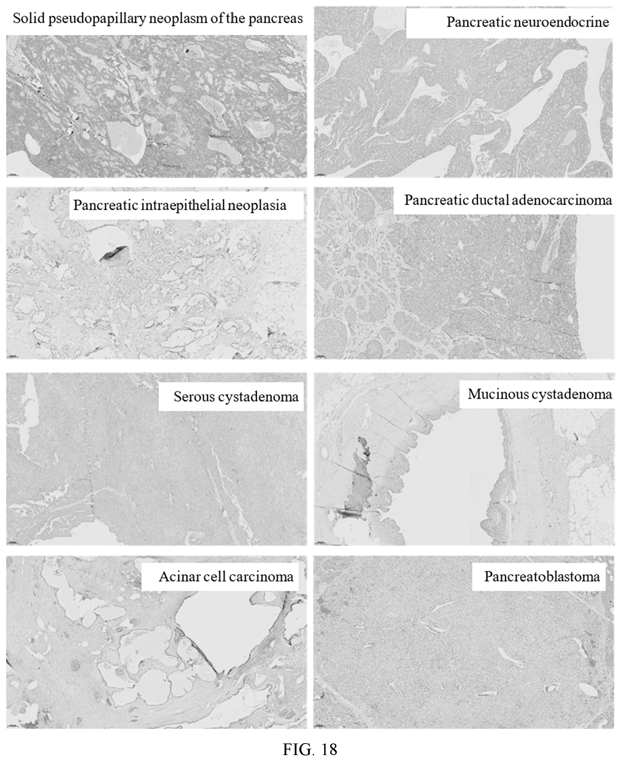

Representative IHC staining images of solid pseudopapillary neoplasm, pancreatic neuroendocrine tumor, pancreatic intraepithelial neoplasia, pancreatic ductal adenocarcinoma, serous cystadenoma, mucinous cystadenoma, acinar cell carcinoma and pancreatoblastoma are shown in FIG. 18.

In FIG. 17, the y-axis represents the percentage of samples with each score relative to the total number of samples in the group. Among 30 cases of solid pseudopapillary neoplasm of the pancreas, 14 cases were scored as ++ and 16 cases were scored as +. Among 20 cases of pancreatic neuroendocrine tumor, 12 cases were scored as - and 8 cases were scored as +. Among 10 cases of pancreatic intraepithelial neoplasia, 3 cases were scored as -. Among 20 cases of pancreatic ductal adenocarcinoma, all 20 cases were scored as -. Among 4 cases of serous cystadenoma, all 4 cases were scored as -. Among 4 cases of mucinous cystadenoma, all 4 cases were scored as -. Among 3 cases of acinar cell carcinoma, all 3 cases were scored as -. Among 3 cases of pancreatoblastoma, all 3 cases were scored as -.

The results of FIG. 17 and FIG. 18 indicate that the anti-ABCD1 antibody prepared in Example 5, when applied for IHC staining combined with manual scoring, can effectively distinguish solid pseudopapillary neoplasm of the pancreas from pancreatic neuroendocrine tumor, pancreatic intraepithelial neoplasia, pancreatic ductal adenocarcinoma, serous cystadenoma, mucinous cystadenoma, acinar cell carcinoma and pancreatoblastoma.

The present application has been described in detail above. It will be apparent to those skilled in the art that the present application can be practiced within a wide range of equivalent parameters, concentrations, and conditions without departing from the spirit and scope of the present application and without undue experimentation. Although the specific embodiments of the present application have been illustrated, it should be understood that further modifications may be made thereto. In general, in accordance with the principles of the present application, the present application is intended to cover any alterations, uses, or improvements thereof, including changes made by conventional techniques known in the art that depart from the scope disclosed in the present application. Applications of the essential features may be made within the scope of the claims.

## Claims

1. An antibody that specifically binds to ATP-binding cassette sub-family D member 1 (ABCD1) or an antigen-binding fragment thereof, wherein the complementarity-determining regions (CDRs) have the following amino acid sequences:
a heavy chain CDR1 selected from at least one of amino acid sequences set forth in SEQ ID NOs: 1-8;
a heavy chain CDR2 selected from at least one of amino acid sequences set forth in SEQ ID NOs: 9-17;
a heavy chain CDR3 selected from at least one of amino acid sequences set forth in SEQ ID NOs: 18-24;
a light chain CDR1 selected from at least one of amino acid sequences set forth in SEQ ID NOs: 25-30;
a light chain CDR2 selected from at least one of amino acid sequences LVS, WAS, RMS or YAS; and
a light chain CDR3 selected from at least one of amino acid sequences set forth in SEQ ID NOs: 31-35.

2. The antibody or the antigen-binding fragment thereof according to claim 1, wherein the amino acid sequences of the CDRs are selected from at least one of the following groups (1)-(10):
(1) the heavy chain CDR1, the heavy chain CDR2, the heavy chain CDR3, the light chain CDR1, the light chain CDR2, and the light chain CDR3 are amino acid sequences set forth in SEQ ID NOs: 1, 9, 18, 25, RMS, and 31, respectively;
(2) the heavy chain CDR1, the heavy chain CDR2, the heavy chain CDR3, the light chain CDR1, the light chain CDR2, and the light chain CDR3 are amino acid sequences set forth in SEQ ID NOs: 2, 10, 19, 26, YAS, and 32, respectively;
(3) the heavy chain CDR1, the heavy chain CDR2, the heavy chain CDR3, the light chain CDR1, the light chain CDR2, and the light chain CDR3 are amino acid sequences set forth in SEQ ID NOs: 3, 11, 20, 27, WAS, and 33, respectively;
(4) the heavy chain CDR1, the heavy chain CDR2, the heavy chain CDR3, the light chain CDR1, the light chain CDR2, and the light chain CDR3 are amino acid sequences set forth in SEQ ID NOs: 1, 12, 18, 25, RMS, and 31, respectively;
(5) the heavy chain CDR1, the heavy chain CDR2, the heavy chain CDR3, the light chain CDR1, the light chain CDR2, and the light chain CDR3 are amino acid sequences set forth in SEQ ID NOs: 1, 13, 18, 25, RMS, and 31, respectively;
(6) the heavy chain CDR1, the heavy chain CDR2, the heavy chain CDR3, the light chain CDR1, the light chain CDR2, and the light chain CDR3 are amino acid sequences set forth in SEQ ID NOs: 4, 14, 21, 25, RMS, and 31, respectively;
(7) the heavy chain CDR1, the heavy chain CDR2, the heavy chain CDR3, the light chain CDR1, the light chain CDR2, and the light chain CDR3 are amino acid sequences set forth in SEQ ID NOs: 5, 15, 22, 28, RMS, and 31, respectively;
(8) the heavy chain CDR1, the heavy chain CDR2, the heavy chain CDR3, the light chain CDR1, the light chain CDR2, and the light chain CDR3 are amino acid sequences set forth in SEQ ID NOs: 6, 16, 23, 29, LVS, and 34, respectively;
(9) the heavy chain CDR1, the heavy chain CDR2, the heavy chain CDR3, the light chain CDR1, the light chain CDR2, and the light chain CDR3 are amino acid sequences set forth in SEQ ID NOs: 7, 17, 24, 30, WAS, and 35, respectively; and
(10) the heavy chain CDR1, the heavy chain CDR2, the heavy chain CDR3, the light chain CDR1, the light chain CDR2, and the light chain CDR3 are amino acid sequences set forth in SEQ ID NOs: 8, 16, 23, 29, LVS, and 34, respectively.

3. The antibody or the antigen-binding fragment thereof according to claim 1 or 2, wherein the heavy chain variable region comprises an amino acid sequence with at least 75%, 80%, 85%, 90%, 95%, 98%, 99%, or 100% identity to any one of the sequences set forth in SEQ ID NOs: 36-45, and the light chain variable region comprises an amino acid sequence with at least 75%, 80%, 85%, 90%, 95%, 98%, 99%, or 100% identity to any one of the sequences set forth in SEQ ID NOs: 46-55.

4. The antibody or the antigen-binding fragment thereof according to any one of claims 1-3, wherein the amino acid sequences of the heavy chain variable region and the light chain variable region are selected from at least one of the following groups (1)-(10):
(1) the heavy chain variable region comprises an amino acid sequence set forth in SEQ ID NO: 36, and the light chain variable region comprises an amino acid sequence set forth in SEQ ID NO: 46;
(2) the heavy chain variable region comprises an amino acid sequence set forth in SEQ ID NO: 37, and the light chain variable region comprises an amino acid sequence set forth in SEQ ID NO: 47;
(3) the heavy chain variable region comprises an amino acid sequence set forth in SEQ ID NO: 38, and the light chain variable region comprises an amino acid sequence set forth in SEQ ID NO: 48;
(4) the heavy chain variable region comprises an amino acid sequence set forth in SEQ ID NO: 39, and the light chain variable region comprises an amino acid sequence set forth in SEQ ID NO: 49;
(5) the heavy chain variable region comprises an amino acid sequence set forth in SEQ ID NO: 40, and the light chain variable region comprises an amino acid sequence set forth in SEQ ID NO: 50;
(6) the heavy chain variable region comprises an amino acid sequence set forth in SEQ ID NO: 41, and the light chain variable region comprises an amino acid sequence set forth in SEQ ID NO: 51;
(7) the heavy chain variable region comprises an amino acid sequence set forth in SEQ ID NO: 42, and the light chain variable region comprises an amino acid sequence set forth in SEQ ID NO: 52;
(8) the heavy chain variable region comprises an amino acid sequence set forth in SEQ ID NO: 43, and the light chain variable region comprises an amino acid sequence set forth in SEQ ID NO: 53;
(9) the heavy chain variable region comprises an amino acid sequence set forth in SEQ ID NO: 44, and the light chain variable region comprises an amino acid sequence set forth in SEQ ID NO: 54; and
(10) the heavy chain variable region comprises an amino acid sequence set forth in SEQ ID NO: 45, and the light chain variable region comprises an amino acid sequence set forth in SEQ ID NO: 55.

5. A polynucleotide, wherein the polynucleotide encodes the antibody or the antigen-binding fragment thereof according to any one of claims 1-4.

6. A recombinant vector, wherein the recombinant vector comprises the polynucleotide according to claim 5.

7. A host cell, wherein the host cell comprises the polynucleotide according to claim 5 and/or the recombinant vector according to claim 6.

8. A pharmaceutical composition, wherein the pharmaceutical composition comprises the antibody or the antigen-binding fragment thereof according to any one of claims 1-4.

9. Use of ABCD1 as a detection marker in any one or more of the following (a)-(c):
(a) use in the development of a diagnostic reagent for diagnosing or assisting in the diagnosis of SPN;
(b) use in the development of a differentiation reagent for differentiating or assisting in the differentiation of SPN from other non-SPN tumors; and/or
(c) use in the development of a screening reagent for screening or assisting in the screening of SPN patients.

10. Use of a substance for detecting ABCD1 in any one or more of the following (a)-(c):
(a) use in the preparation of a diagnostic reagent for diagnosing or assisting in the diagnosis of SPN;
(b) use in the preparation of a differentiation reagent for differentiating or assisting in the differentiation of SPN from other non-SPN tumors; and/or
(c) use in the preparation of a screening reagent for screening or assisting in the screening of SPN patients.

11. Use of a substance for detecting ABCD1 in the preparation of a kit, wherein the kit is configured for any one or more of the following (a)-(c):
(a) for diagnosing or assisting in the diagnosis of SPN;
(b) for differentiating or assisting in the differentiation of SPN from other non-SPN tumors; and/or
(c) for screening or assisting in the screening of SPN patients.

12. The use according to any one of claims 10-11, wherein the substance for detecting ABCD1 comprises an anti-ABCD1 antibody, an ABCD1 gene-specific primer and an ABCD1 gene-specific probe.

13. The use according to any one of claims 10-12, wherein the substance for detecting ABCD1 comprises the antibody or the antigen-binding fragment thereof according to any one of claims 1-4.

14. The use according to any one of claims 10-13, wherein the diagnostic reagent, the differentiation reagent, or the screening reagent further comprises a substance for detecting one or more of the following markers: β-catenin, CD10, vimentin, and α1-antitrypsin.

15. The use according to any one of claims 9-11, wherein the other non-SPN tumors comprise pancreatic acinar cell carcinoma, pancreatoblastoma, pancreatic neuroendocrine tumor, and pancreatic ductal adenocarcinoma; and the pancreatic neuroendocrine tumor comprises non-functional pancreatic neuroendocrine tumor and insulinoma.

16. The use according to any one of claims 9-11, wherein
the object of diagnosis or assistance in diagnosis using the diagnostic reagent is a tumor, and the object of differentiation or assistance in differentiation using the differentiation reagent is a tumor, preferably a pancreatic tumor; and/or
the subject for screening or assistance in screening using the screening reagent is a tumor patient, preferably a tumor sample of a pancreatic tumor patient.

17. A method for diagnosing or assisting in the diagnosis of SPN, comprising: using ABCD1 as a marker and detecting the marker for diagnosing or assisting in the diagnosis of SPN.

18. The method according to claim 17, wherein detecting the marker comprises:
performing ABCD1 protein mass spectrometry analysis, qPCR detection, transcriptome sequencing, and/or ABCD1 immunostaining on a clinical sample.

19. The method according to claim 18, wherein the immunostaining comprises immunohistochemical staining, immunocytochemical staining, immunofluorescence staining, and/or multiplex immunofluorescence staining.

20. The method according to claim 18, wherein the ABCD1 immunostaining is performed using the antibody or the antigen-binding fragment thereof according to any one of claims 1-4.

21. A method for differentiating or assisting in the differentiation of SPN from other non-SPN tumors, comprising: using ABCD1 as a marker and detecting the marker for differentiating or assisting in the differentiation of SPN from other non-SPN tumors.

22. The method according to claim 21, wherein the other non-SPN tumors comprise pancreatic acinar cell carcinoma, pancreatoblastoma, pancreatic neuroendocrine tumor, and pancreatic ductal adenocarcinoma; and the pancreatic neuroendocrine tumor comprises non-functional pancreatic neuroendocrine tumor and insulinoma.

23. The method according to claim 21, wherein detecting the marker comprises:
performing ABCD1 protein mass spectrometry analysis, qPCR detection, transcriptome sequencing, and/or ABCD1 immunostaining on a clinical sample.

24. The method according to claim 23, wherein the immunostaining comprises immunohistochemical staining, immunocytochemical staining, immunofluorescence staining, and/or multiplex immunofluorescence staining.

25. The method according to claim 23, wherein the ABCD1 immunostaining is performed using the antibody or the antigen-binding fragment thereof according to any one of claims 1-4.
